# EUROPEAN PATENT SPECIFICATION

(11) **EP 1 581 812 B1**
(45) Date of publication and mention of the grant of the patent: **16.07.2008**
(21) Application number: 04700319.9
(22) Date of filing: 06.01.2004
(51) Int. Cl.: G01N 33/574

(54) **COMPOSITIONS AND METHODS FOR DIAGNOSING AND TREATING COLON CANCERS**
ZUSAMMENSETZUNGEN UND VERFAHREN ZUR DIAGNOSE UND BEHANDLUNG VON KOLONKREBS
COMPOSITIONS ET PROCEDE PERMETTANT DE DIAGNOSTIQUER ET DE TRAITER LES CANCERS DU COLON

(30) Priority: 06.01.2003 US 438000 P
(43) Date of publication of application: 05.10.2005
(73) Proprietor: Wyeth, Madison, New Jersey 07940 (US)
(72) Inventor: MARTINEZ, Robert, Vincent, Roslindale, MA 02131 (US); BROWN, Eugene, Newton Highlands, MA 02461 (US); LIU, Wei, Sudbury, MA 01776 (US)
(74) Representative: Denholm, Anna Marie
(86) International application number: PCT/US2004/000035
(87) International publication number: WO 2004/061423

(56) References cited:
- EP-A- 1 602 930
- WO-A-20/04005457
- WO-A-20/04074436
- WO-A-20/04098521
- WO-A-20/05040828
- WO-A1-99/48921
- YAMAMOTO Y ET AL: "OVEREXPRESSION OF ORPHAN G-PROTEIN-COUPLED RECEPTOR, GPR49, IN HUMAN HEPATOCELLULAR CARCINOMAS WITH BETA-CATENIN MUTATIONS" HEPATOLOGY, WILLIAMS AND WILKINS, BALTIMORE, MD, US, vol. 37, no. 3, March 2003 (2003-03), pages 528-533, XP008041122 ISSN: 0270-9139
- HSU ET AL MOL. ENDOCRINOL. vol. 12, no. 12, 1998, pages 1830 - 1845

## Description

### TECHNICAL FIELD

The present invention relates generally to the diagnosis of colon cancer. The invention specifically relates to colon cancer genes that are differentially expressed in colon cancer tissues as compared to disease-free tissues. These genes can be used for prognosing or diagnosing colon cancer.

### BACKGROUND OF THE INVENTION

Cancer is a significant health problem throughout the world. Although advances have been made in detecting and treating cancer, no vaccine or other universally successful method for prevention or treatment is currently available. Current therapies, which are generally based on a combination of chemotherapy or surgery and radiation, continue to prove inadequate in many patients.

Colon cancer is the second most frequently diagnosed malignancy in the United States, as well as the second most common cause of cancer death. An estimated 135,400 new cases of colon cancer were diagnosed in 2001, with an estimated 56,700 deaths. The five-year survival rate for patients with colon cancer detected in an early localized stage is 92%; unfortunately, only 37% of colon cancer is diagnosed at this stage. The survival rate drops to 64% if the cancer is allowed to spread to adjacent organs or lymph nodes, and to 7% in patients with distant metastases.

The prognosis of colon cancer is directly related to the degree of penetration of the tumor through the bowel wall and the presence or absence of nodal involvement; consequently, early detection and treatment are especially important. Colon cancer typically originates in the colonic epithelium and is not extensively vascularized (and therefore not invasive) during the early stages of development. The transition to a highly-vascularized, invasive and ultimately metastatic cancer commonly takes ten years or longer. With early detection and diagnosis, colon cancer may be effectively treated by, for example, surgical removal of the cancerous or precancerous tissue. However, colon cancer is often detected only upon manifestation of clinical symptoms, such as pain and black tarry stool. Generally, such symptoms are present only when the disease is well established, and only after metastasis has occurred. Early detection of colon cancer is therefore important in order to significantly reduce its morbidity. Currently, the best means of preventing colon cancer is through early detection of pre-neoplastic lesions in the colon through various invasive and noninvasive screening techniques.

Most methods for colon cancer screening are invasive. Invasive diagnostic screening methods, such as endoscopic examination, allow for direct visual identification, removal, and biopsy of potentially-cancerous tissue. However, invasive cancer screening procedures are often expensive, inherently risky, and can result in severe medical complications. Invasive screening procedures also frequently result in significant patient discomfort. The discomfort associated with typical invasive screening methods reduces patient compliance with routine screening procedures. For example, flexible sigmoidoscopy is an invasive procedure for diagnosing colon cancer that enables detection of approximately 55% of all colon cancer and is estimated to have an 85% sensitivity with a near 100% specificity. However, the procedure has a complication rate of about 4.5 per 10,000 persons screened. Further, patient compliance with physicians' recommendations to undergo sigmoidoscopy is low, reportedly varying from 30 to 75%, due to discomfort and perceived embarrassment associated with this procedure.

Non-invasive methods of colon cancer screening involve assaying samples for the presence of materials that are indicative of cancer or pre-cancer. Established non-invasive methods for detection of colon cancer focus on extracellular indicia of the presence of cancer, such as the presence of fecal occult blood or elevated levels of carcinoembryonic antigen, both of which are suggestive of the presence of colon cancer. However, such extracellular indicia typically occur only after the cancer has become invasive, and therefore more difficult to treat. As a result, many non-invasive screening procedures are of limited value in the early diagnosis of cancer. For example, fecal occult blood testing (FOBT) is a non-invasive screening test for colon cancer that is highly variable in accuracy, ranging between 28% and 93%, depending upon the subject's hydration status, with a specificity of 96%. One study estimates, however, that 50 to 60% of all colorectal cancers will be missed if FOBT is the only method of screening used (Allison et al., Ann. Intern. Med., 112:328-333, 1990).

Recent developments in molecular biology provide methods of great potential for detecting the presence of a range of DNA mutations indicative of oncogenesis. Mutations and the loss of heterozygosity at the p53 tumor suppressor locus have been correlated with various types of cancer. The loss or other mutation of the APC and DCC tumor suppressor genes has also been associated with tumor development. It has been suggested that specific mutations might be a basis for molecular screening assays for the early stages of certain types of cancer. Accordingly, non-invasive screening assays that are highly sensitive and highly specific for detecting the presence of a range of DNA mutations indicative of cancer have been developed. For instance, the presence of such mutations can be detected in DNA found in stool samples during various stages of colon cancer.

Treatment regimens are determined by the type and stage of the cancer, and include surgery, radiation therapy or chemotherapy. Recurrence following surgery (the most common form of therapy) is a major problem and is often the ultimate cause of death. Current methods for prognosing, detecting and treating colon cancer have failed to provide satisfactory results for reducing the morbidity associated with the disease.
WO2004/005457 discloses strong expression of GPR47 in colon cancer cells.

### SUMMARY OF THE INVENTION

The present invention relates to a method of detecting colon cancer, comprising the steps of: detecting a level of a GPR49 polypeptide or the expression profile of the GPR49 gene in a biological sample of a subject; and comparing said level to a control level of said GPR49 polypeptide or control expression profile; wherein the biological sample is a colon tissue sample and the control level is an average level of said polypeptide in control samples of disease-free subjects; and wherein differential expression of the GPR49 gene is used to indicate the presence of colon cancer. In one embodiment, the colon cancer genes are differentially expressed not only between colon cancer tissues and disease-free colon tissues, but also between colon cancer tissues and one or more other disease-free tissues. These other disease-free tissues include, but are not limited to, cervix, kidney, left atrium, left ventricle, right atrium, right ventricle, lung, ovary, prostate, rectum, skin, or stomach. Differential expression can be either over-expression or under-expression.

In another embodiment, the colon cancer genes are over-expressed in colon cancer tissues relative to disease-free colon tissues. The average expression levels of these genes in colon cancer tissues can be, for example, at least 1.5, 2, 3, 4, 5, 10, 20, or more times of those in disease-free colon tissues. In many cases, the p-value of the differential expression analysis for each selected colon cancer gene is no more than 0.1, 0.05, 0.001, 0.0005, 0.0001, or less.

The present invention is useful for diagnosing or monitoring colon cancer in a subject of interest. The methods include the steps of detecting the levels of a GPR49 polypeptide or the expression profile of the GPR49 gene in a biological sample of the subject, and comparing the detected levels to control levels. The biological sample is a colon tissue sample. The control levels are average levels of the GPR49 polypeptide or the expression profile of the GPR49 gene in control samples of disease-free subjects. In another embodiment, the biological sample and the control samples are prepared using the same procedure. In yet another embodiment, the levels of the GPR49 polypeptide or the expression profile of the GPR49 gene are determined by using antibodies specific for the polypeptides. The subject of interest may or may not have colon cancer. In one embodiment, the subject has colon cancer and is subject to a therapeutic treatment of the cancer. In another embodiment, the subject is a human, a canine, or another mammal.

The present invention is useful for diagnosing or monitoring colon cancer in a subject of interest. The methods include the steps of detecting the expression profile of one or more colon cancer genes in a biological sample of the subject, and comparing the expression profile to a control expression profile The expression profile and the control expression profile can be determining by measuring the levels of the polypeptides or polynucleotides encoded by the one or more colon cancer genes. In one embodiment, the control expression profile is an average expression profile of the one or more colon cancer genes in controls samples of disease-free subjects.

### BRIEF DESCRIPTION OF THE DRAWINGS

The inventions of this application are better understood in conjunction with the following drawing. The drawing is provided for illustration, not limitation.

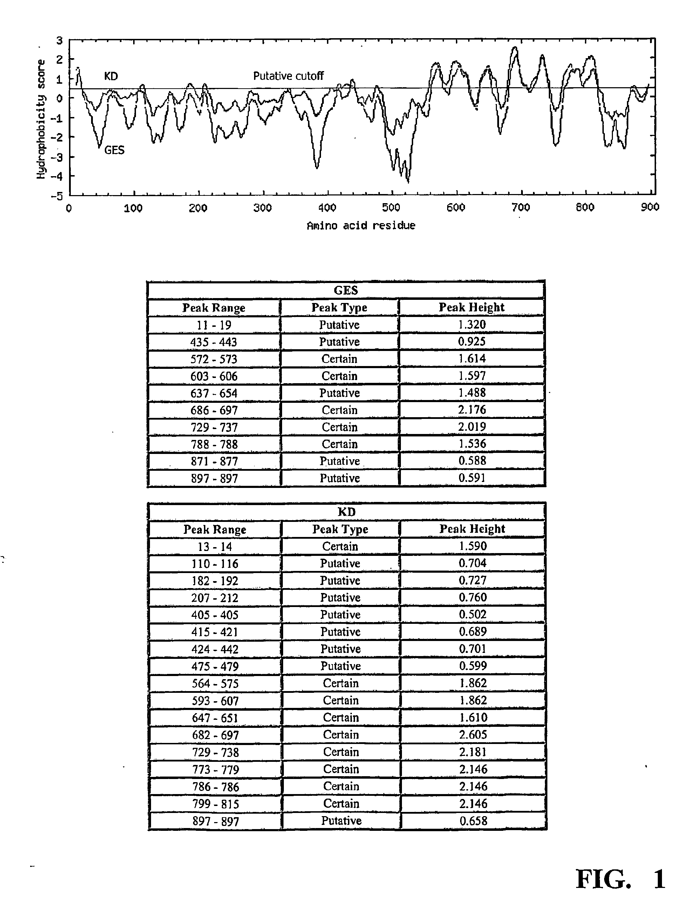

**FIG. 1** depicts the hydrophobicity profile of the polypeptide consisting of an amino acid sequence recited in SEQ ID NO:84.

### DETAILED DESCRIPTION OF THE INVENTION

Various aspects of the invention are described in further detail in the following subsections. In this application, the use of "or" means "and/or" unless stated otherwise.

### Colon Cancer Genes (CCGs)

The present invention concerns methods of using a colon cancer gene (CCGs) for the prognosis or diagnosis of colon cancer. A colon cancer gene is a gene that is differentially expressed in colon cancer cells as compared to disease-free colon cells. Specifically, the present invention relates to a method of detecting colon cancer, comprising the steps of: detecting a level of a GPR49 polypeptide or the expression profile of the GPR49 gene in a biological sample of a subject; and comparing said level to a control level of said GPR49 polypeptide or control expression profile; wherein the biological sample is a colon tissue sample and the control level is an average level of said polypeptide in control samples of disease-free subjects; and wherein differential expression of the GPR49 gene is used to indicate the presence of colon cancer. In a particular embodiment, the subject has been subjected to a therapeutic treatment of colon cancer.

In one embodiment, the colon cancer gene is differentially expressed not only between colon cancer tissues and disease-free colon tissues, but also between colon cancer tissues and one or more other disease-free tissues. These other disease tissues include, for example, cervix, kidney, left atrium, left ventricle, right atrium, right ventricle, lung, ovary, prostate, rectum, skin, and stomach tissues. In many examples, the p-value of the differentiation expression analysis for each selected colon cancer gene is no more than 0.1, 0.05, 0.001, 0.0005, 0.0001, or less.

In another embodiment, the colon cancer gene is over-expressed in colon cancer tissues relative to one or more disease-free tissues. In certain cases, the average expression level of the colon cancer gene in colon cancer cells is at least 0.5, 1, 2, 3, 4, 5, 10, 20, or more times higher than that in disease-free colon cells.

GPR49 is over-expressed in colon cancer cells by at least 2-fold as compared to disease-free colon cells.
The GPR49 polypetptide employed in the present invention is a receptor, illustrated in Table 1.

| Table 1 Group III genes: Receptors | | | |
|---|---|---|---|
| Gene symbol | Locus link. | Nucleic acid seq. | Amino acid seq. |
| GPR49 | 8549 | SEQ ID NO:21 | SEQ ID NO:84 |

GPR49 (G protein-coupled receptor 49) is an orphan-G protein-coupled receptor with an unknown ligand. Expression of GPR49 gene has been reported in brain, skeletal muscle, placenta, and spinal cord. The hydrophobicity profile of GPR49 is shown in FIG. 1.

### CCGs and CCG Products as Markers for Colon Cancer

CCGs, their encoded polynucleotides (CCPNs), and polypeptides (CCPPs) can be used as markers for colon cancer. The CCG of the present invention is GPR47.

The expression levels of CCGs are correlated with the presence of colon cancer. In certain embodiments, the present invention can be performed by detecting the presence of CCPNs or CCPPs using any suitable method known in the art. In another aspect, the expression levels of the CCGs are determined in a biological sample of a particular subject for which either diagnosis or prognosis information is desired according to claim 1. The expression profile of one or more CCGs can be used as a "fingerprint" to represent the disease state of a cell. In some examples, relative levels of expression are indicative of the severity of colon cancer and as such, can be used for diagnostic and prognostic analyses. Moreover, by comparing relative expression profiles of CCGs from tissue samples taken at different points in time, e.g., pre- and post-therapy or at different time points within a course of therapy or during colon cancer development, information regarding which gene is important for each of these stages can be obtained. In one example, comparison of expression profiles of CCGs at different stages of the tumor progression provides a method for long-term prognosis, including survival. In another example, a particular treatment regime can be evaluated based on CCG expression profiles, including whether a particular drug will act to improve the long-term prognosis in a particular patient.

The discovery of the differential expression patterns for individual or panels of CCGs allows for screening for test compounds that modulate a particular expression pattern. For example, screening can be done for compounds that will convert an expression profile for a poor prognosis to one for a better prognosis. This can be done by making biochips comprising sets of the significant CCGs, which can then be used in these screens. These methods can also be done on the protein level. Protein expression levels of the CCGs can be evaluated for diagnostic and prognostic purposes, or used to screen test compounds. For example, significant CCGs can comprise CCGs which are determined to have modulated activity or expression in response to a therapy regime. Alternatively, the modulation of the activity or expression of a CCG can be correlated with the diagnosis or prognosis of colon cancer.

### Sources of CCG Products

Polynucleotides and polypeptides encoded by CCGs (*i.e.,* CCPNs and CCPPs, respectively) can be isolated from any suitable tissue or cell of a subject of interest. The biological example of the present invention is colon tissue. In addition, CCPNs or CCPPs can be prepared by using, without limitation, nucleic acid amplification, recombinant vectors encoding CCGs, chemical synthesis, or other methods as appreciated by those skilled in the art.

### Detection Methods

As discussed earlier, expression level of CCGs may be used as a marker for colon cancer. Detection and measurement of the relative amount of a CCG product (polynucleotide or polypeptide) of the invention can be carried out by any method known in the art.

Typical methodologies for detection of a transcribed polynucleotide include RNA extraction from a cell or tissue sample, followed by hybridization of a labeled probe (*e.g.,* a complementary polynucleotide molecule) specific for the target RNA to the extracted RNA and detection of the probe (*e.g.,* Northern blotting).

Typical methodologies for peptide detection include protein extraction from a cell or tissue sample, followed by binding of an antibody specific for the target protein to the protein sample, and detection of the antibody. Antibodies can be detected by the use of a labeled secondary antibody. The label can be a radioisotope, a fluorescent compound, an enzyme, an enzyme co-factor, or ligand. Such methods are well understood in the art.

In certain embodiments, the CCGs themselves may serve as markers for colon cancer. For example, an increase of genomic copies of a CCG, such as by duplication of the gene, may also be correlated with colon cancer.

Detection of specific polynucleotide molecules may also be assessed by gel electrophoresis, column chromatography, or direct sequencing, quantitative PCR (in the case of polynucleotide molecules), RT-PCR, or nested-PCR among many other techniques well-known to those skilled in the art.

Detection of the presence or number of copies of all or a part of the CCG of the invention may be performed using any method known in the art. Typically, it is convenient to assess the presence or quantity of a DNA or cDNA by Southern analysis, in which total DNA from a cell or tissue sample is extracted, is hybridized with a labeled probe (e.g., a complementary DNA molecules), and the probe is detected. The label group can be a radioisotope, a fluorescent compound, an enzyme, or an enzyme co-factor. Other useful methods of DNA detection or quantification include direct sequencing, gel electrophoresis, column chromatography, and quantitative PCR, as is known by one skilled in the art.

In certain embodiments, the CCPPs may serve as markers for colon cancer. Detection of specific polypeptide molecules may be assessed by gel electrophoresis, Western blot, column chromatography, or direct sequencing, among many other techniques well-known to those skilled in the art.

### Determining severity of colon cancer

In the field of diagnostic assays, the invention is useful for determining the severity of colon cancer by isolating a sample from a subject (e.g., a colon biopsy), detecting the presence, quantity or activity of the CCG of the invention in the sample relative to a second sample from a normal sample or control sample. In one embodiment, the expression levels of the CCG in the two samples are compared, and a modulation in one or more CCGs in the test sample indicates colon cancer. In other embodiments, modulations of 2, 3, 4 or more CCGs indicate a severe case of colon cancer.

An example agent for detecting CCPP is an antibody capable of binding to CCPP. In one example, the antibody is conjugated with a detectable label. Antibodies can be polyclonal or monoclonal. An intact antibody, or a fragment thereof (e.g., Fab or F(ab')₂) can be used. The term "labeled," with regard to the probe or antibody, is intended to encompass direct labeling of the probe or antibody by coupling (*e.g.,* physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.
That is, the detection method of the invention can be used to detect CCG mRNA, protein or genomic DNA in a biological sample *in vitro* as well as *in vivo.* For example, *in vitro* techniques for detection of CCG mRNA include Northern hybridizations and *in situ* hybridizations. *In vitro* techniques for detection of CCPP include enzyme linked immunosorbent assays (ELISAs), Western blots, immunoprecipitations and immunofluorescence. *In vitro* techniques for detection of CCG genomic DNA include Southern hybridizations. Furthermore, *in vivo* techniques for detection of CCPP include introducing into a subject a labeled anti-CCPP antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject.

In another embodiment, the methods further involve obtaining a control biological sample from a subject, contacting the control sample with a compound or agent capable of detecting CCG protein, mRNA, or genomic DNA, such that the presence of CCG protein, mRNA or genomic DNA is detected in the biological sample, and comparing the presence of CCG protein, mRNA or genomic DNA in the control sample with the presence of CCG protein, mRNA or genomic DNA in the test sample.

It should be understood that the above-described embodiments and the following examples are given by way of illustration, not limitation. Examples

### Example 1: Identification of colon cancer genes

A query was performed to identify genes that are uniquely over-expressed in colon adenocarcinoma tissue relative to adjacent normal tissues using GeneExpress Oncology DataSuite™ and the fold change analysis function within GX2000 analytical program (Gene Logic Inc., Gaithersburg, MD). The GeneExpress Oncology DataSuite™ is an interactive information system that provides the global gene expression profiles of human cancer types. The GeneExpress system is based on the collection of cancer and normal tissue samples, the generation of global gene expression data using high-density microarrays, and the analysis of results using a variety of sophisticated software tools, such as GX2000. The normal tissues include human colon, cervix, kidney, left atrium, left ventricle, right atrium, right ventricle, lung, ovary, prostate, rectum, skin and stomach.

Initially, a total of 495 genes were found to have a two-fold differential expression between normal and cancer tissue. Contrast analysis was then used on these 495 genes to identify those genes having a p-value equal to or lower than 0.05. The contrast analysis generated 429 genes. Upon visual inspection of these 429 genes using e-northems, 63 genes were identified as uniquely over-expressed in colon cancer tissue relative to the panel of normal tissues described above These 63 genes were defined as colon cancer genes (CCGs). One of these, GPR47, is shown in table 9

### Example 2: Hydrophobicity analysis

The hydrophobicity profiles of the polypeptides encoded by the CCGs were generated using the TopPred II program (Claros et al., TopPred II: An Improved Software For Membrane Protein Structure Predictions., CABIOS, 10, 685-686, 1994) at the Bioweb site maintained by the Pasteur Institute (Paris, France). The hydrophobicity profile is demonstrated in both KD (Kyte and Doolittle) scale and GES (Goldman, Engelman and Steitz) scale.

Briefly, KD scale is a hydropathy scale that associated a hydropathy value to each amino acid. A moving-window approach is implemented in which the hydrophobicity scale over a number of adjacent residues in the native sequence is summed in order to identify membrane regions. A threshold value T is defined to label a segment as 'membrane helix': if the sum over the hydrophobicity exceeded T, the segment was predicted to be a membrane helix.

The GES scale is also used to identify nonpolar transbilayer helices. The curve is the average of a residue-specific hydrophobicity scale over a window of 20 residues. When the line is in the upper half of the frame (positive), it indicates a hydrophobic region and when it is in the lower half (negative), a hydrophilic region.

In a typical hydrophobicity profile, the X-axis represents the length of the protein in amino acids (aa), while the Y-axis represents the KD or GES score. The curve line shows the KD or GES pattern of the entire protein, while the strait line shows putative cutoffs for potential membrane spanning domains.

## Claims

1. A method of detecting colon cancer comprising the steps of: detecting an expression profile of the GPR49 gene by measuring a level of a GPR49 polypeptide or polynucleotide in a colon tissue sample of a subject, and comparing said level to a control level, wherein differential expression of the GPR49 gene is used to indicate the presence of colon cancer.

2. A method as claimed in claim 1, wherein the subject has been subjected to a therapeutic treatment of colon cancer.

## Patentansprüche

1. Verfahren zur Erkennung von Kolonkrebs, umfassend folgende Schritte: Erkennen eines Expressionsprofils des GPR49-Gens durch Messen eines Niveaus eines GPR49-Polypeptids oder -Polynukleotids in einer Kolongewebeprobe eines Probanden, und Vergleichen des Niveaus mit einem Kontrollniveau, wobei die differentielle Expression des GPR49-Gens verwendet wird, um die Anwesenheit von Kolonkrebs anzuzeigen.

2. Verfahren nach Anspruch 1, wobei der Proband einer therapeutischen Behandlung von Kolonkrebs ausgesetzt wurde.

## Revendications

1. Procédé de détection du cancer du colon comprenant les étapes de : détection d'un profil d'expression du gène GPR49 par mesure d'un taux de polypeptide GPR49 ou de polynucléotide GPR49 dans un échantillon de tissu de colon d'un sujet, et la comparaison dudit taux à un taux de référence, dans lequel l'expression différentielle du gène GPR49 est utilisée pour indiquer la présence du cancer du colon.

2. Procédé tel que revendiqué selon la revendication 1, dans lequel le sujet a été soumis à un traitement thérapeutique du cancer du colon.
